(19) Europäisches Patentamt
European Patent Office
Office européen des brevets

(11) Veröffentlichungsnummer: **0 059 974**
**B1**

(12) **EUROPÄISCHE PATENTSCHRIFT**

(45) Veröffentlichungstag der Patentschrift: **31.07.85**

(51) Int. Cl.⁴: **C 07 D 473/08**

(21) Anmeldenummer: **82101867.8**

(22) Anmeldetag: **09.03.82**

(54) **Verfahren zur Herstellung von Phenoxyalkancarbonsäureestern von Hydroxyäthyltheophyllin.**

(30) Priorität: **11.03.81 DE 3109291**

(43) Veröffentlichungstag der Anmeldung: **15.09.82 Patentblatt 82/37**

(45) Bekanntmachung des Hinweises auf die Patenterteilung: **31.07.85 Patentblatt 85/31**

(84) Benannte Vertragsstaaten: **AT BE CH DE FR GB IT LI LU NL SE**

(56) Entgegenhaltungen:
**EP - A - 0 049 494**
**DE - A - 2 308 826**
**DE - A - 2 461 069**
**US - A - 2 575 344**

**ARZNEIMITTELFORSCHUNG, Band 30(II), Nr. 11b, 1980, Seiten 2014-19, Aulendorf (DE); G. METZ et al.: "Zur Chemie der Phenoxyessigsäureester von Oxyalkyltheophyllinen und 1-(Theophyllin-7-yl)-äthyl-2-(2-(p-chlorphenoxy)-2-methylpropionat) (Etofyllinclofibrat), einem neuen Antilipämikum"**

(73) Patentinhaber: **Ludwig Merckle GmbH & Co. chem.-pharm. Fabrik, Dr.-Georg-Spohn-Strasse 7, D-7902 Blaubeuren (DE)**

(72) Erfinder: **Metz, Gunter, Dr., Auf dem Rucken 29, D-7902 Blaubeuren (DE)**
Erfinder: **Specker, Manfred, Dr., Weilerhalde 32, D-7902 Blaubeuren-Weiler (DE)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Gegenstand der Erfindung ist ein Verfahren zur Herstellung von Phenoxyalkancarbonsäureestern von Hydroxyäthyltheophyllin (Etofyllin) der allgemeinen Formel I

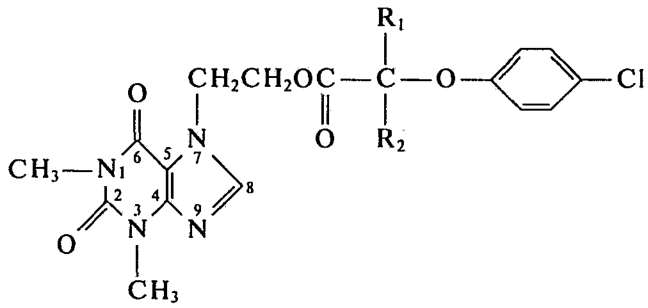

(I)

in der $R_1$ und $R_2$ unabhängig voneinander jeweils ein Wasserstoffatom oder einen $CH_3$-Rest bedeuten. Nach dem Verfahren bringt man einen Halogenäthylester der allgemeinen Formel II

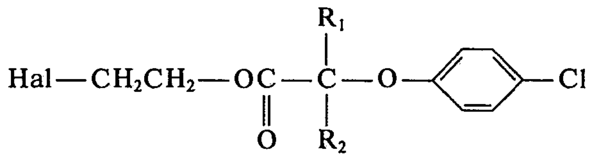

(II)

in der $R_1$ und $R_2$ obige Bedeutung besitzen, und Hal ein Chlor- oder Bromatom bedeutet, mit einem Natrium- oder Kaliumsalz von Theophyllin in einem aprotischen Lösungsmittel direkt zur Reaktion. Geeignete aprotische Lösungsmittel sind Acetonitril und Hexamethylphosphorsäuretriamid (HMPT) sowie vorzugsweise Dimethylformamid.

Ester der allgemeinen Formel I sind aus der DE-PS 23 08 826 bekannt. Von ihnen zeigt insbesondere der Ester der Clofibrinsäure ($R_1 = R_2 = CH_3$) mit Etofyllin (Etofyllinclofibrat) besondere pharmakologische Eigenschaften, die therapeutisch nutzbar sind. Das in der DE-PS 23 08 826 beschriebene Verfahren zur Herstellung der Ester der allgemeinen Formel I benutzt reaktionsfähige Derivate der Phenoxyalkancarbonsäuren insbesondere deren Säurechloride, die unter Zusatz von Hilfsbasen mit Etofyllin in geeigneten Lösungsmitteln zur Reaktion gebracht werden. Dieses Verfahren beruht auf einer gängigen Darstellungsmethode von Estern, zeigt aber im technischen Herstellungsprozeß erhebliche Mängel, insbesondere in bezug auf Synthese und Handhabung der entsprechenden Säurechloride. Phenoxyalkancarbonsäurechloride sind relativ instabile, nicht lagerungsfähige Verbindungen. Bei ihrer technischen Herstellung durch Umsatz mit $SOCl_2$, $POCl_3$ oder $PCl_5$ entstehen große Mengen von gasförmigem HCl, die nur mit besonderem technischem Aufwand beseitigt werden können. Hinzu kommt ferner, daß diese Säurechloride unter Autokatalyse zur Zersetzung neigen, dadurch in größeren Mengen nicht destilliert bzw. gereinigt werden können und somit als Rohsäurechloride eingesetzt werden müssen.

Der Einsatz dieser Rohsäurechloride führt je nach Restanteil an nicht umgesetzten $SOCl_2$, $POCl_3$ oder $PCl_5$ bei der Reaktion mit Etofyllin zu Nebenprodukten, die nur schwer durch Kristallisation abtrennbar sind. Die durch Reaktion mit den Rohsäurechloriden hergestellten Ester der allgemeinen Formel I erfordern weiterhin zur Reinigung und Abtrennung gefärbter Verunreinigungen eine zweifache Kristallisation.

Wie nun überraschend gefunden wurde, können diese Verfahrensnachteile durch das erfindungsgemäße Verfahren behoben werden. Zudem werden die Ester überraschenderweise in insgesamt höherer Endausbeute und hoher Reinheit erhalten. Ein weiterer Vorteil dieses Verfahrens besteht darin, daß sowohl die Halogenäthylderivate der allgemeinen Formel II wie auch die Natrium- und Kaliumsalze von Theophyllin stabile, lagerfähige Derivate sind, die ein technisch vorteilhaftes Einstufenverfahren erlauben.

Das erfindungsgemäße Verfahren benutzt aprotische Lösungsmittel bei Reaktionstemperaturen von etwa 50°C bis zum Rückfluß des Lösungsmittels.

Unter den gängigen aprotischen Lösungsmitteln haben sich HMPT (Hexamethylphosphorsäuretriamid) und DMF (Dimethylformamid) als besonders vorteilhaft erwiesen. Die erforderlichen Reaktionszeiten betragen zwischen 2 und 6 Stunden. Durch Verdünnen mit oder Eingießen in Wasser fallen die Ester in so hoher Reinheit an, daß eine getrennte Kristallisation nicht erforderlich ist. Für technische Ansätze ist es günstig, nach Abschluß der Reaktion das verwendete Lösungsmittel überwiegend durch Vakuumdestillation zurückzugewinnen und im Destillationsrückstand durch Zugabe von Wasser oder Alkohol die gewünschten Ester auszufällen bzw. zu suspendieren. Zur Endreinigung der so erhaltenen Ester genügt es, das Produkt mit einem geeigneten Lösungsmittel, wie Äthanol oder Isopropanol, zur Entfernung eventuell vorhandener nicht-wasserlöslicher Ausgangsstoffe kalt auszuwaschen bzw. beim

Absaugen nachzuwaschen. Die verwendeten Ausgangsstoffe werden vorzugsweise in äquimolaren Mengen umgesetzt, da ein Überschuß an einem der Reaktanten nur unwesentlich höhere Ausbeute erbringt.

Es ist überraschend, daß jeweils nur das Halogenatom an der Äthylgruppe des Halogenäthylesters der allgemeinen Formel II mit dem Alkalimetallatom in Reaktion tritt, so daß die gewünschte Verbindung in überraschend hoher Ausbeute anfällt, ohne daß die zu erwartenden Nebenreaktionen mit dem Halogenatom am Phenoxyrest in die Ausbeute beeinträchtigender Weise eintreten.

Die hohe Esterausbeute ist auch deshalb besonders überraschend, als sie unter Einsatz nicht wasserfreier d. h. technischer Lösungsmittel erzielt wird und unter den benutzten Reaktionsbedingungen beim Einsatz basischer Alkalimetallsalze bzw. deren Hydrate mit wesentlicher Ausbeuteminderung infolge von Hydrolyse zu rechnen war. Hinzu kommt ferner, daß die Alkalimetallsalze untrennbare Reste von Alkalihydroxyd aus dem Herstellungsprozeß enthalten und dadurch bevorzugt höhermolekulare und gegen Hydrolyse besonders empfindliche Ester verseifen können.

Das erfindungsgemäße Verfahren ist in folgenden Beispielen näher erläutert.

Beispiel 1

21,8 g (0,1 Mol) Theophyllinkalium werden mit 27,7 g (0,1 Mol) β-Chloräthylclofibrinsäureester in 140 ml DMF 3 Stunden auf 120°C erhitzt. Aus dem Ansatz werden im Wasserstrahlvakuum ca. 100 ml DMF abdestilliert. Der Eindampfrückstand wird mit 500 ml Wasser unter kräftigem Rühren versetzt. Das farblose Kristallisat wird abgesaugt und mit 50 ml Äthanol nachgewaschen. Man erhält 34,1 g (81,0%) Etofyllinclofibrat vom Fp 135—136°C.

Beispiel 2

22,0 g (0,1 Mol) Theophyllinnatrium-Monohydrat werden mit 27,7 g (0,1 Mol) β-Chloräthyl-clofibrinsäureester in 50 ml HMPT 3 Stunden bei 60—65°C umgesetzt. Das Lösungsmittel wird im Wasserstrahlvakuum abdestilliert und der Rückstand mit 100 ml Äthanol versetzt und kräftig gerührt. Das Kristallisat wird abgesaugt, mit 100 ml Wasser und anschließend mit 30 ml Äthanol nachgewaschen. Man erhält 37,6 g (89,4%) Etofyllinclofibrat vom Fp 134—135°C.

Folgende weitere Ester der allgemeinen Formel I wurden entsprechend diesem Verfahren hergestellt:

| Beispiel Nr. | $R_1$ | $R_2$ | Verfahren gemäß Beispiel | Lösungsmittel | Temp. °C | Reaktionszeit (Std.) | Ausbeute % | Fp. °C |
|---|---|---|---|---|---|---|---|---|
| 3 | H | H | 1 | HMPT | 60–65 | 2,5 | 86,2 | 120–121 |
| 4 | H | H | 2 | DMF | 90–100 | 3 | 85,1 | 120 |
| 5 | $CH_3$ | H | 2 | DMF | 80 | 3 | 80,4 | 117–118 |
| 6 | $CH_3$ | H | 1 | DMF | 90 | 3 | 83,1 | 117 |

**Patentanspruch**

Verfahren zur Herstellung von Phenoxyalkancarbonsäureestern von Etofyllin der allgemeinen Formel I

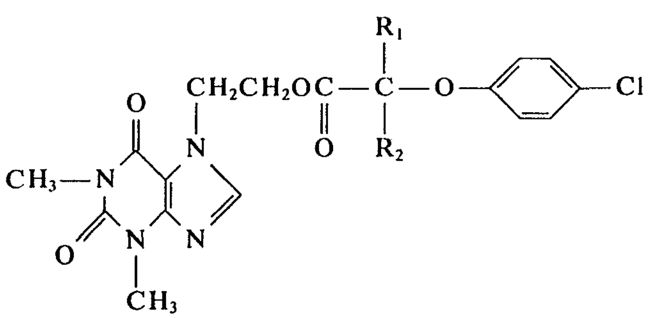

(I)

in der $R_1$ und $R_2$ unabhängig voneinander jeweils ein Wasserstoffatom oder einen $CH_3$-Rest bedeuten, dadurch gekennzeichnet, daß man einen Halogenäthylester einer Phenoxyalkancarbonsäure der allgemeinen Formel II

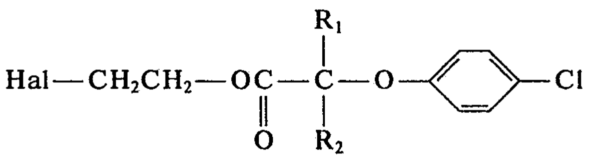

(II)

in der $R_1$ und $R_2$ obige Bedeutung besitzen, und Hal ein Chlor- oder Bromatom bedeutet, mit einem Natrium- oder Kaliumsalz von Theophyllin in einem aprotischen Lösungsmittel bei Reaktionstemperaturen zwischen 50°C und Rückflußtemperatur umsetzt.

## Claim

Process for the production of phenoxyalkane carboxylic acid esters of etofylline of the general formula I

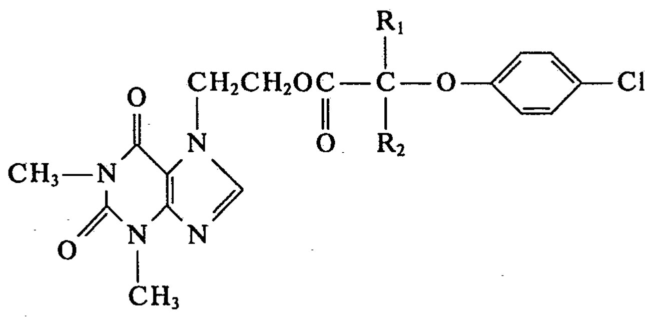

(I)

in which $R_1$ and $R_2$ are each separately a hydrogen atom or a $CH_3$ radical, characterized in that a halogen ethyl ester of a phenoxyalkane carboxylic acid of the general formula II

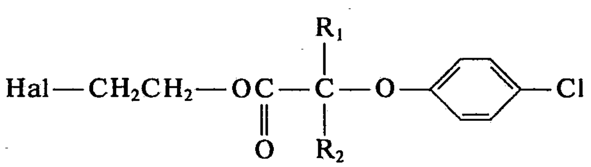

(II)

in which $R_1$ and $R_2$ have the above meaning and Hal is a chlorine atom or bromine atom, is reacted with a sodium salt or potassium salt of theopylline in an aprotic solvent at reaction temperatures of between 50°C and reflux temperature.

## Revendication

Procédé pour la préparation de phénoxyalcane-carboxylates d'étofylline de formule générale I

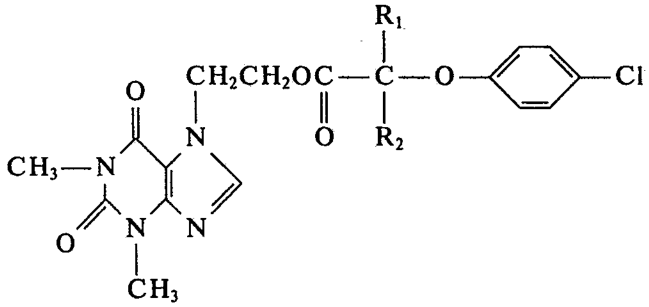

(I)

dans laquelle $R_1$ et $R_2$, indépendamment l'un de l'autre, représentent chacun un atome d'hydrogène ou un radical $CH_3$, caractérisé en ce qu'on fait réagir un ester éthylique halogéné de formule générale II

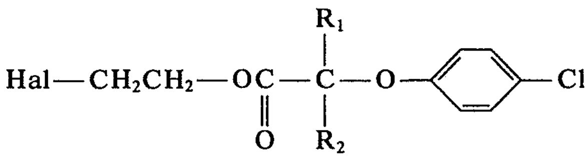

(II)

dans laquelle $R_1$ et $R_2$ ont les significations données ci-dessus et Hal représente un atome de chlore ou de brome, avec un sel sodique ou potassique de théophylline dans un solvant aprotique, à des températures de réaction comprises entre 50°C et la température de reflux.